# EUROPEAN PATENT APPLICATION

(11) **EP 4 613 243 A1**
(43) Date of publication of application: **10.09.2025**
(21) Application number: 24162044.2
(22) Date of filing: 07.03.2024
(51) Int. Cl.: A61F 2/38

(54) **A CERAMIC FEMORAL COMPONENT OF A KNEE PROTHESIS**

(71) Applicant: CeramTec GmbH, 73207 Plochingen (DE)
(72) Inventor: BISSCHOP, Bastian, 73033 Göppingen (DE); GEBERT DE UHLENBROCK, Anne, 91367 Weißenohe (DE); UPMANN, Carsten, 73730 Esslingen (DE); MAIER, Dennis, 73312 Geislingen (DE); HÄUßLER, Kim-Lars, 70176 Stuttgart (DE)
(74) Representative: Maikowski & Ninnemann Patentanwälte Partnerschaft mbB

(57) **Abstract**

The present invention relates to a ceramic femoral component (200) of a knee prothesis comprising
- an inner surface of the femoral component, and
- at least one, preferably at least two projections (207a, 207b) extending inwardly away from the inner surface of the femoral component for guiding the ceramic femoral component on the patient's femur in anterior-posterior direction and positioning the ceramic femoral component on the patient's femur by formfit or pressfit.

## Description

The present invention relates to a ceramic femoral component of a knee prothesis.

Joint replacements, in particular artificial knee joint replacements have been known for quite some time. Such a knee system includes a tibial component, a femoral component, and optionally a patellar component.

The femoral component is connected with the distal end of the femur of the patient. The femoral component typically has inner surfaces that face and are at least partially in contact with a surgically prepared distal femoral bone, and an outer surface with a curvature that faces the corresponding tibial component (tray and insert), in particular the insert, which is attached to the patient's tibia by the tibial tray.

The different components of the knee prothesis (femoral, tibial, patella) are made of metal, polymeric, or ceramic materials. Depending on the material used for the prothesis, there are cemented and uncemented systems. For example, a femoral component made of metal can be fitted onto the femur of a patient via press-fit or via a transitional fit which typically do not require additional cement for attaching the component on the patient's femur. In case of a femoral component made of ceramic a loose fit or clearance fit is preferred due to the specific properties of the ceramic material that require cemented system.

Bone from the distal femur of the patient must be removed to fit and accommodate the femoral component. During knee surgery, the femur bone is cut with the help of sawing templates and cutting guides. Before the final femoral implant component is inserted, the positioning of the femoral component onto the bone cut is checked by using a femoral trial

The outer bearing geometry of such a femoral trial typically reflects the geometry of the final femoral implant.

When using a metal femoral trial and a metal femoral component, geometrical deviations are only minor, since both can be placed on the patient's femur by press fit, in particular by an anterior-posterior press fit. Due to the properties of metal, a press fit of both trial implant and final implant is permissible and not critical in terms of component safety,

However, when using a femoral trial made of metal or plastic and a femoral implant made of ceramic material, geometrical deviations between femoral trial and final femoral implant may be encountered. While the outer geometries of femoral trial and femoral implant may be identical, the inner geometries deviate. This is due to the circumstance that a ceramic femoral component cannot be placed on the patient's femur by large press fit, but preferably by a loose fit or clearance fit, as mentioned above.

Depending on the geometry, ceramic implants can react extremely sensitively to tensile loading, i.e. when designing a ceramic femoral implant, the implant must not be allowed to expand due to press fit while attempting to place the final femur implant component on the patient's femur. Thus, spreading or expansion of ceramic prothesis when fitting the ceramic prothesis on the prepared femur of the patient should be avoided due to the sensitivity of the ceramic material to tensile load.

Any extensive tensile load on the ceramic femoral component can be avoided by designing the saw cut of the patient's femur smaller than the internal or inner geometry of the femoral component such there is no or only a slight interference fit between patient's femur and femoral component. The smaller design of the patient's femur in comparison to the femoral component creates, however, a gap between the processed patient's femur and the ceramic femoral component. The size of the gap may be between 1 mm and 3 mm.

On the contrary, the trial is designed smaller than the internal geometry of the femoral component. The correct saw cut or saw design of the patient's femur is therefore checked using the ideally fitting femoral trial with a slight press fit for avoiding a falling off, for example a press-fit between I2I and 1 mm.

This is followed by placing the final ceramic femoral component on the patient's femur.

However, the difference between geometry of femoral bone and femoral prosthetic component creates a gap preventing a transitional fit and allowing for a movement in anterior - posterior direction or tilting in flexion of the femoral prosthetic component. This is not desirable.

The object of the present invention was therefor to provide a femoral prosthetic component that avoids such a movement or tilting.

This object is solved by a femoral prosthetic component according to claim 1.

Accordingly, a ceramic femoral component of a knee prothesis with an anterior end and a posterior end is provided, wherein the femoral component comprises
- an inner surface of the femoral component, and
- at least one, preferably at least two projections extending inwardly away from the inner surface of the femoral component for guiding the ceramic femoral component on the patient's femur in anterior-posterior direction and positioning the ceramic femoral component on the patient's femur by formfit or pressfit.

By providing a femoral component with formfitting projections, preferably for loose fit or clearance fit, on the inner surface of the ceramic femoral component it is possible to guide, position and immobilize the ceramic femoral component on the patient's femur in a manner that a movement or tilting of the ceramic femoral prosthetic component in anterior- posterior direction is avoided or at least reduced to a limited clearance.. Within the meaning of the present invention, "formfit" is a form of fastening effected by geometrical contact of two effective areas.

By providing a femoral component with pressfitting projections (such as fins) on the inner surface of the ceramic component it is possible to exert a press fit between ceramic femoral component and patient's femur for positioning and anchoring the ceramic femoral component on the patient's femur. A "press fit" or "friction fit" is a form of fastening between two tightfitting mating parts that produces a joint which is held together by contact pressure an/or friction after the parts are pushed together.

The formfitting or pressfitting projections of the present ceramic femoral component may have different shapes depending on their arrangement on the inner surface of the femoral component.

It is to be understood that femoral components with stems as projections are generally known. For example, in WO 2017/149279 A1 a femoral component made of a polymeric material is described that comprises conical stems extending inwardly away from the internal surface of the femoral component for engaging corresponding sockets formed in the patient's femur.

However, conical stems as described for femoral components made of polymeric material are not suitable for femoral components made of ceramic material as in the present case. As pointed out above, in comparison to other materials ceramic material has a lower tensile load. A stand-alone conical stem as described in WO 2017/149279 A1 in a ceramic femoral component would therefore be prone for breaking. Furthermore, such stems are not applicable as means for guiding the femoral component in anterior-posterior direction and positioning the femoral component on the patient's femur.

Besides, stand-alone projections, in particular stand-alone (narrow or slim) conical stems, in combination with an intercondylar box are not suitable for (ceramic) femoral implants and should therefore be avoided. This is in particular since said intercondylar box is responsible for medial - lateral positioning of the femoral component.

It is rather preferred that the formfitting or pressfitting projections of the present ceramic femoral component provide a stable system wherein the formfitting or pressfitting projections are not prone to break, in particular that the projections are more stable compared to stand-alone stems or alike as described in the prior art for other prosthesis materials.

The ceramic material used for preparing the present femoral component is generally known (see US 2011/0251698 A1, WO 2016/102976 A1). Examples of suitable ceramic materials include alumina, zirconia, zirconia toughened and silicon nitrides.

Before discussing the invention in more detail, the general structure of a femoral component is described.

The inner surface of the femoral component (i.e. inner side of the femoral component) includes five portions: an anterior portion (i.e. the portion on the inner surface of the femoral component that is opposite to the patella shield on the outer surface of the femoral component), an anterior chamfer portion, a distal portion, a posterior chamfer portion, a posterior portion.

The five different portions of the inner surface of the femoral component contact each other along a contact line or bending line in predetermined angles. The contact angle of the different portions on the inner surface of the femoral component is typically about 135°.

As will become more apparent further below, the anterior portion of the inner surface of the femoral component may further be divided into a (lower) section adjacent to contact line with the chamfer anterior portion and an upper section.

The patient's distal femur is resected or cut in such a way as to include corresponding resected surfaces that are shaped to receive the inner surface of prosthetic femoral component with the respective five portions. Specifically, distal femur includes anterior resected surface that is shaped to receive anterior portion of the inner surface of the femoral component, anterior chamfer resected surface that is shaped to receive anterior chamfer portion of the inner surface of the femoral component, distal resected surface that is shaped to receive distal portion of the inner of the femoral component, posterior chamfer resected surface that is shaped to receive posterior chamfer portion of the inner surface of the femoral component, and posterior resected surface that is shaped to receive posterior portion of the inner surface of the femoral component.

It is to be noted that not all of the five portions of the present ceramic femoral component are in contact with the femur bone when positioned on the femur bone. For example, while the distal portion may be in contact with the femur bone, the anterior portion and posterior portion may not be in contact with the femur bone, thus the cementing of the present ceramic femoral component may be required.

Furthermore, the distal portion of the inner surface of the femoral component comprises typically a recess on both of the outer edges of the distal portion. Said recesses are used for engaging with operational or surgical instruments for moving and positioning the femoral component on the resected femur of the patient.

The femoral component has typically a medial condyle section and a lateral condyle section with a trochlear groove or patella groove joining the first and second condyle section. Each of the medial condyle section and the lateral condyle section has respective distal region or portion i.e. a distal medial condyle portion and a distal lateral condyle portion. Distal medial condyle portion and distal lateral condyle portion are disposed from each other to form an intracondylar space or recess or notch. The patella groove is spanning over the first and second condyle section to said intracondylar space.

Medial condyle section and lateral condyle section of the femoral component are bone contacting surface that rests against the patient's resected distal femur. The respective distal regions or condyle portions articulate against the tibial component of the knee prothesis, i.e. distal regions engage a portion of a tibial component of a knee prothesis.

An intercondylar box may be disposed between the distal medial condyle portion and distal lateral condyle portion for filling the intercondylar space between the distal medial condyle portion and distal lateral condyle portion. Said box includes a medial box wall and a lateral box wall, wherein the walls extend from the inner surface of the femoral component towards an upper end / upper edge of said wall. The intercondylar box is described in more detail further below.

As mentioned previously, the formfitting or pressfitting projections of the present ceramic femoral component are provided and arranged on inner the surface of the femoral component. In particular, the formfitting or press fitting projections of the present femoral component may be provided in different locations or portions of the inner surface of the femoral component.

Thus, in an embodiment of the ceramic femoral component of a knee prothesis the at least one, preferably at least two press fitting projections (or fins) extending inwardly from the inner surface of the ceramic femoral component are provided in the anterior portion of the inner surface of the femoral component. This embodiment will be described in detail further below as a second aspect of the invention.

In another embodiment of the ceramic femoral component according to a first aspect of the invention the at least one, preferably at least two formfitting projections are provided in the distal portion of the inner surface of the femoral component. In this case, the projections are preferably arranged in in the distal medial condyle portion and/or the distal lateral condyle portion of the femoral component.

More specifically, the formfitting projections in the distal portion of the inner surface of the ceramic femoral component are provided as an integral part of said medial and lateral box walls of the intercondylar box disposed between the distal medial condyle portion and distal lateral condyle portion for filling the intercondylar space.

Thus, according to an embodiment of this first aspect of the invention, a ceramic femoral component of a knee prothesis with an anterior end and a posterior end is provided, wherein the ceramic femoral component comprises
- an surface of the femoral component including an anterior portion, an anterior chamfer portion, a distal portion, a posterior chamfer portion and a posterior portion,
- a medial condyle section with a distal medial condyle portion,
- a lateral condyle section with a distal lateral condyle portion,
- an intercondylar space between the distal medial condyle portion and the distal lateral condyle portion,
- an intercondylar box substantially disposed between the distal medial condyle portion and distal lateral condyle portion for filling the intercondylar space, the box including a medial box wall and a lateral box wall, the walls extending from the inner surface of the femoral component towards an upper end or upper edge of said walls,
- wherein each of the medial box wall and the lateral box wall comprise at least one projection (or protrusion) for engaging into corresponding sockets formed in a patient's femur for guiding the ceramic femoral component on the patient's femur in anterior-posterior direction and positioning the femoral component on the patient's femur by formfit or slight press-fit,
- wherein each of the at least one projections extends inwardly from the distal portion of the inner surface of the femoral component along the respective medial box wall and the lateral box wall towards the upper end (or edge) of the medial box wall and the lateral box wall.

The projections are an integral part of the respective wall, in particular said projections are formed as one-piece with the respective medial box wall and lateral box wall.

In an embodiment, said box wall projections have an angular or cylindrical shape, in particular a semi-cylindrical shape, with a tapered, conical (or round off) tip, in particular semi-conical tip, towards the upper end of the respective box wall. In particular, the round off tip may aid the insertion and positioning of the projections into the sockets or boreholes formed in the patient's femur. The projections may have also any other suitable shape enabling a guiding and positioning of the femoral component on the patient's femur, such as star-shaped, triangular, rectangular, for example with rounded off edges.

In another embodiment, said box wall projections (or positioning elements) have a diameter between 2 and 10 mm, preferably between 3 and 8 mm, more preferably between 4 and 6 mm.

The length of the box wall projections depends on the height of the box walls which is depending on the implant size. Typically, the height of the box wall and said box wall projections may be between 2 and 15 mm, preferably between 4 and 15 mm, more preferably between 7 and 11 mm.

It is further of an advantage, if the said box wall projections are provided with an offset directed outwards away from the respective box wall allowing a better handling for the medical personnel.

In a further embodiment, said box wall projections extend inwardly from the distal portion of the innersurface of the femoral component along and integrated into the medial box wall and lateral box wall towards the upper end (or edge) of the medial box wall and the lateral box wall. i.e. the box wall projections are formed as one piece with the respective box wall.

The location of the projections in respect to the inner surface of the femoral component depends on the shape of the box. For example, said box wall projections may be arranged in the center of the distal portion of the inner surface of the femoral component. It is also possible that the box wall projections are centered over the contact point of the femoral component with the tibial component when the contact point is 0° flexion (full extension) in vivo as defined in DIN EN ISO 21536 (The flexion-extension range of angular movement between the femoral and tibial components of a total or uni-compartmental knee joint replacement shall include angles from less than or equal to 0° flexion to a maximum greater than or equal to 110° flexion. Angular measurements shall be made with a tolerance of ±1°.

In another embodiment of the femoral component, a cam portion may be disposed between the distal medial condyle portion and distal lateral condyle portion. Embodiments can include one cam, preferably posterior cam or can include two cams, a posterior cam and anterior cam be formed as independent structures extending between the medial and lateral condyle portions of the femoral component, while other cams may be a portion of the femoral component forming a boundary of the intercondylar space, such as the edge of the femoral component forming the anterior boundary of the intercondylar space.

Said cam can comprise a variety of configurations, shapes and dimensions. In some embodiments" one or more cams may substantially comprise a cylindrical shape including, for example, an elliptic, oblique, parabolic, or hyperbolic cylinder. In other embodiments, one or more cams may comprise irregular cross-sections, which can include one or more curvilinear and/or straight portions or sides. In a preferred embodiment the cam is a posterior cam provided with a cylindric shape having notches. Said notches are preferably as small as possible to have sufficient flexural strength, but also allow for freedom of rotation in deep flexion.

As mentioned previously, the intercondylar box is disposed between the distal medial condyle portion and distal lateral condyle portion for filling the intercondylar space or notch. The width of the box comprises the distance from at least a portion of the medial box wall to at least a portion of the lateral box wall. At least a portion of the medial box wall and/or a portion of the lateral box wall has a defined box-wall thickness. The box has preferably a symmetrical configuration.

The intercondylar box including the medial box wall and the lateral box wall may comprise a variety of configurations, shapes and dimensions. For example, the medial box wall and a lateral box wall of the intercondylar box can be curved, oblique, straight or a combination thereof, preferably curved. Specifically, the box may comprise curved walls (or surfaces) that are curved in one, two or three dimensions, planar walls (or surfaces) that are oblique in one, two or three dimensions. Curved designs are possible in any desired direction and in combination with any planar or oblique planar surfaces. Thus, the box can be round and square for positioning on the femur between the joint rollers by transition fit, press fit or clearance fit.

In general, the dimensions of the box depend on the width and depth of the intercondylar space wherein the box width is based, at least in part on patient specific information, and wherein the patient specific information comprises one or more patient-specific dimensions selected from one of: mediolateral length of the femur, a mediolateral length of the tibia, and a distance from a medial-most point of a medial epicondyle of the femur to a lateral-most point of a lateral epicondyle of the femur.

In some embodiments, the box and/or cam(s) of the femoral component may be configured to engage a post projecting from a tibial implant component (e g., tibial. polyethylene insert).

In some embodiments, the box, and/or cam(s) are configured to allow M-L (medial-lateral) rotation of the femoral component relative to the tibial component through at least a portion of flexion and/or extension.

In an embodiment, the present femoral component comprises cement pockets arranged in the inner surface of femoral component and configured to receive cement for anchoring the ceramic femoral component on the patient's femur.

The present femoral component may comprise at least one, preferably at least two, more preferably at least three cement pockets arranged in the anterior portion of the inner surface of the femoral component (said cement pockets may also be named anterior cement pockets).. In an embodiment, three separate cement pockets are provided in the anterior portion, preferably one larger cement pocket and two smaller cement pockets. By providing two smaller cement pockets in addition to a larger cement pocket, the anchoring of the femoral component is improved, in particular in cases where the patella shield is larger than the femur bone cut. Besides, the cement pockets add to the fixation of the femoral component on the femur bone. As described previously, the anterior portion of the inner surface of the femoral component may be divided into a (lower) section adjacent to the chamfer anterior portion and a (distal) upper section. Anterior portion and chamfer anterior portion of the inner surface of the femoral component are arranged to each other in an angel (like 135°) along a contact line or bending line. In an embodiment, the two smaller cement pockets are provided in the (lower) section of the anterior portion that is adjacent to the chamfer anterior portion and the larger cement pocket is provided in the upper section of the anterior portion.

In an embodiment, the cement pockets are provided in the anterior portion of the inner surface of the femoral component in the shape of a half-arch or semi-circular arch. In the preferred case of three separate cement pockets, the half-arch is divided into three separate cement pockets, wherein two (smaller) cement pockets form each the distal or end regions of a half-arch and one (larger) cement pocket forms the middle region of the half-arch.

In a further embodiment, further (additional) cement pockets are provided on the lateral and medial condyle spanning over anterior chamfer portion, distal portion, posterior chamfer portion and posterior portion, respectively. In a preferred embodiment, two additional cement pockets are arranged in the posterior portion of the inner surface of the femoral component (said cement pockets may also be named posterior cement pockets). In another embodiment, two additional cement pockets span over anterior chamfer portion, distal portion and posterior chamfer portion (said cement pockets may also be named distal cement pockets). Separate cement pockets may also be provided in the posterior portion.

The cement pockets may have a depth between 0.5 and 2.0 mm, preferably between 1.0 mm and 1.5 mm. The volume of the cement pocket depends on the location on the inner surface of the femoral component (i.e. distal or anterior condyle portion), as well as on the size of the femoral component and thus on the specific patient parameters. The volume of the cement pockets may vary between 200 and 2000 mm³, preferably between 500 and 1500 mm³. The volume of the cement pockets in the anterior condyle portion may be between 200 and 800 mm³, while the volume of the cement pockets in the distal portion may be between 700 and 2000 mm³.

As mentioned previously, the femur of the patient has to be prepared for receiving the ceramic femoral component. For this purpose, a femoral cut guide is provided to prepare the patient's distal femur to receive the prosthetic femoral component. More particular, a cut guide is provided to remove bone from distal femur to accommodate the femoral component. The femoral trial may serve as a trial or provisional component used to possibly reduce and test the prepared knee just before implanting the final prosthetic femoral component. Besides, the femoral trial guides the femoral component in anterior-posterior direction.

In the present case, the femoral trial is similar (but not identical) in size and shape to prosthetic femoral component of the knee prosthesis. In particular, femoral trial and ceramic femoral component have a different inner geometry due to the specific mechanical properties of the ceramic material, as mentioned previously.

The femoral trial may include an articulating or outer surface that is substantially identical in size and shape to articulating surface of prosthetic femoral component, medial and lateral condyles that are substantially identical in size and shape to medial and lateral condyles of prosthetic femoral component, and a cam that is substantially identical in size and shape to the cam of the prosthetic femoral component.

Also, the femoral trial may include an inner surface that includes anterior portion, anterior chamfer portion, distal portion, posterior chamfer portion and posterior portion.

However, the inner geometry of the femoral trial is smaller than the inner geometry of the femoral component. The smaller geometry of the femoral trial allows for a press fitting of 0-2 mm on the patient's femur. In contrast, the inner geometry of the ceramic femoral component allows for a transitional fit of 0-2 mm on the patient's femur.

Furthermore, the geometry of the respective portions of the femoral trial and the ceramic femoral component deviate such that for example the distal portion of the femoral trial has no bone contact to the femur bone, while the ceramic femoral component has bone contact.

In addition, the femoral trial comprises through holes, preferably at least one or two through holes that allow for providing holes in the distal femur of the patient for guiding and receiving the box wall projections of the present femoral component as positioning elements. The position of the through holes in the trial implant corresponds to the position of the box wall projections in the ceramic femoral component. The diameter of through holes is larger than the diameter of the semicircular box wall projections by about 0.1 to 0.8 mm, preferably about 0.2 to 0.6 mm, more preferably about 0.3 to 0.5 mm, such as approximately 0.4 mm.

As indicated previously, in a second aspect of the invention thepress fitting projections of the present femoral component may be also be provided in the anterior portion of the inner surface of the femoral component.

Thus, according to an embodiment of a second aspect of the invention, a ceramic femoral component of a knee prothesis with an anterior end and a posterior end is provided, wherein the ceramic femoral component comprises
- an inner surface of the femoral component comprising at least an interior portion, in particular an anterior portion, an anterior chamfer portion, a distal portion, a posterior chamfer portion and a posterior portion, and
- at least one, preferably at least two projections (or protrusions or fins) extending inwardly from the anterior portion of the inner surface of the femoral component for engaging into the patient's femur for positioning the femoral component on the patient's femur by pressfit.

It is to be understood that the ceramic femoral component of this second embodiment comprises also a medial condyle section with a distal medial condyle portion, a lateral condyle section with a distal lateral condyle portion and an intercondylar space between the distal medial condyle portion and the distal lateral condyle portion, as described in detail above.

In the embodiment according to the second aspect of the ceramic femoral component, said (anterior) projections or fins extending from the anterior portion (or patella portion) of the ceramic femoral component bridge or span over the previously described gap between the femur bone and the femoral component (the gap being due to the different inner geometry of the trial implant and the femoral component).

Furthermore, the projections extending from the anterior portion of the ceramic femoral component press into the bone of the patient's femur; i.e. said projections exert a press fit between ceramic femoral component and patient's femur for positioning and anchoring the ceramic femoral component on the patient's femur. By exerting a press-fit any moving or tilting of the ceramic femoral component is reduced or avoided.

As mentioned previously, the anterior portion of the inner surface of the femoral component may be divided into a (lower) section adjacent to the chamfer anterior portion and a (distal) upper section. In an embodiment, said (anterior) projections are provided in the lower section of the anterior portion; i.e. in close proximity to the contact line (or bending line) between the anterior portion and the chamfer anterior portion.

In an embodiment, said (anterior) projections are provided in the anterior portion of the inner surface of the femoral component perpendicular (or vertically) to said contact line of the anterior portion and the anterior chamfer portion.

Furthermore, said (anterior) projections are provided in a section of the anterior portion of the inner surface of the femoral component that is opposite to the previously described patella groove joining the first and second condyle section of the outer surface of the femoral component.

The femoral component of the second aspect may also comprise cement pockets, as discussed previously. Thus, three separate cement pockets may be provided in the anterior portion, preferably one larger cement pocket and two smaller cement pockets, wherein the two smaller cement pockets are provided in the lower section of the anterior portion that is adjacent to the chamfer anterior portion and the larger cement pocket is provided in the upper section of the anterior portion.

In a preferred embodiment, said (anterior) projections extending inwardly from the anterior portion of the femoral component are provided in the lower section of the anterior portion adjacent to the chamfer anterior portion of the inner surface of the femoral component between cement pockets, in particular between two cement pockets; i.e. the (anterior) projections are centered between the two (smaller) cement pockets provided in the lower section of the anterior portion of the ceramic femoral component.

In another embodiment of the femoral component of the second aspect, the anterior portion may comprise only one cement pocket that is provided in the upper section of the anterior portion. Said cement pocket reflects the middle region or section of a half-arch. The two smaller cement pockets provided in the lower section of the anterior portion are omitted in this embodiment. Rather, the two smaller cement pockets are replaced by (anterior) projections extending inwardly from the anterior portion; i.e. said (anterior) projections are provided in the lower section of the anterior portion as well, but are moved from the more central region of the lower section to the outer region of the lower section.

In an embodiment, the projections in the anterior projections or fins of the femoral component have a height between 0.5 mm and 2.5 mm, preferably between 1.00 mm and 2.0 mm, more preferably between 1.2 mm and 1.5 mm and a length between 5 and 50 mm, preferably between 10 and 40 mm, more preferably between 20 and 30 mm, and a width between 0.5 and 5.0 mm, preferably between 0.7 and 4.0 mm, more preferably between 1.0 mm and 2.5 mm.

In a preferred embodiment, the projections or fins are designed to press or intrude into the femur bone. For example, the fins may have a tapered edge continuing over the entire length of the fin. For example, the projection or fin may have the shape of a three-sided prism.

The femoral component according to the second aspect may also comprise a cam substantially disposed between the distal medial condyle portion and distal lateral condyle portion. Further details of the cam are described previously and are fully applicable.

Furthermore, in one embodiment the femoral component according to the second aspect may also comprise an intercondylar box substantially disposed between the distal medial condyle portion and distal lateral condyle portion for filling the intercondylar space, the box including a medial box wall and a lateral box wall, the walls extending from the inner surface of the femoral component towards an upper end / upper edge of said wall. Further details of the intercondylar box are described previously and are fully applicable.

If the femoral component comprises both anterior projections (or fins) and an intercondylar box it is important to note that the box would have to contact the femur bone ahead of the fins (i.e. box contacts femur bone first, and fins contact femur bone afterwards). This ensures that alignment of the femoral component in medial-lateral (ML) direction is guided by the box, in particular by a high square shaped box.

However, it is preferred and conceivable that in an embodiment of the femoral component according to the second aspect an intercondylar box substantially disposed between the distal medial condyle portion and distal lateral condyle portion for filling the intercondylar space is omitted; i.e. the intercondylar space is free of any box including any box walls.

The ceramic femoral component according to the present invention is described in the following in more detail by means of example with reference to the accompanying drawings. It shows:
- Figure 1 A-C: a schematic side view of a femoral component fit onto a patient's femur;
- Figure 2 A: a schematic side view of a femoral component according to a first embodiment;
- Figure 2 B: a schematic perspective view of the femoral component according to a first embodiment of Figure 2B;
- Figure 3 A-B: schematic perspective views of a trial implant for a femoral component according to a first embodiment of Figures 2A, 2B;
- Figure 4 A-D: schematic views of a femoral component according to a second embodiment; and
- Figure 5: a schematic perspective view of a femoral component according to a third embodiment.

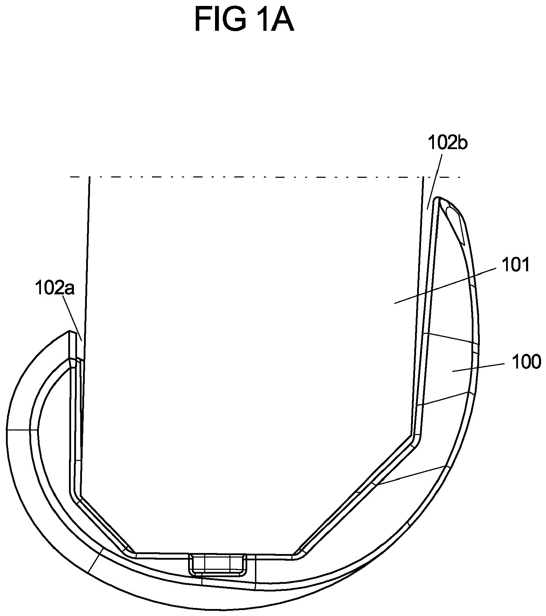

Figures 1 A-C show a femoral component 100 connected with the distal end of the femur 101 of the patient. The femoral component 100 typically has inner surfaces that face and are in contact with a surgically prepared distal femoral surface.

Bone from the distal femur 101 of the patient must be removed from to fit and accommodate the femoral component 100. During knee surgery, the femur bone is cut or resected with the help of sawing templates.

Before the final femoral implant component 100 is inserted, the bone saw cut is checked by using a trial implant. The articulating geometry of such a trial or guide implant typically reflects the geometry of the final femoral implant 100. However, when using a final femur implant component 100 made of ceramic material, geometrical deviations between trial implant and final femoral implant need to be taken into consideration.

Ceramic material is known to be sensitive to high tensile load. When fitting a femoral component made of ceramic material onto the femur any spreading or expansion of the femoral component should be avoided. This can be achieved by providing a ceramic femoral component with a somewhat larger internal geometry compared to the resected femur surface. The smaller resected area of the patient's femur in comparison to the femoral component creates gaps 102a,b between the processed patient's femur and the femoral prosthetic component. The size of the gaps 102a,b may be between 1 and 3 mm, such as 1 mm on the posterior section and 1.8 mm on the anterior section of the ceramic femoral component.

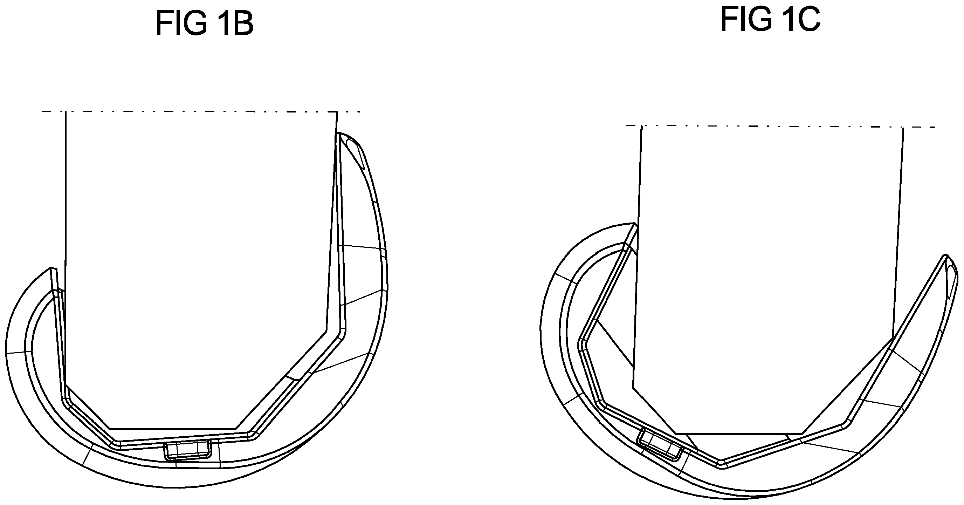

However, this gap 102 between femur 101 and femoral prosthetic component 100 allows for a movement in anterior- posterior direction or tilting of the femoral prosthetic component for flexion or extension (see Figures 1B, 1C).

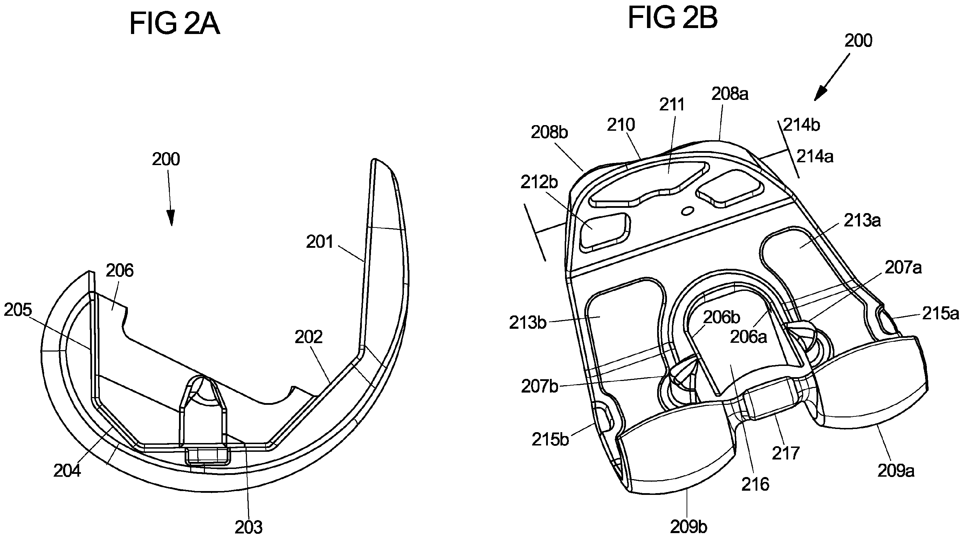

In order to avoid such a movement or tilting of the femoral component on the patient's femur, the femoral component is provided with projections extending inwardly away from the inner surface of the femoral component for positioning and anchoring the femoral component on the patient's femur as illustrated in Figures 2A-B, 4A-D and 5.

Figures 2A, 2B show a femoral component 200 with an inner surface on the inner side of the femoral component: The inner surface of the femoral component 200 includes five portions or regions: anterior portion 201, anterior chamfer portion 202, distal portion 203, posterior chamfer portion 204, posterior portion 205.

The five different portions 201-205 of the inner surface of the femoral component contact each other along contact line or bending line in predetermined angles. For example anterior portion 201 and anterior chamfer portion 202 are arranged adjacent to each other in angel of 135°. The same applies to the arrangement of distal portion 203, posterior chamfer portion 204, posterior portion 205, respectively.

The anterior portion 201 may further be divided into a lower section 214a adjacent to contact line with the chamfer anterior portion 202 and an upper section 214b.

The distal portion 203 comprises two recesses 215a, 215b on each outer side or outer edge of the distal portion 203 for engaging with operational or surgical instruments for moving and positioning the femoral component on the resected femur of the patient.

As common, the femoral component 200 has a medial condyle section 208a and a lateral condyle section 208b with a trochlear or pattella groove 210 joining the first and second condyle section. The medial condyle section 208a has a distal medial condyle portion 209a, and the lateral condyle section 208b has a distal lateral condyle portion 209b. Distal medial condyle portion 209a and distal lateral condyle portion 209b are disposed from each other to form an intracondylar space 216.

The intracondylar space 216 is filled by means of an intercondylar box 206 disposed between the distal medial condyle portion 209a and distal lateral condyle portion 209b. Intercondylar box 206 includes a medial box wall 206a and a lateral box wall 206b, wherein the walls 206a, 206b extend from the inner surface of the femoral component towards an upper end / upper edge of the walls 206a, 206b. The box wall has a curved shape.

Cylindrically shaped projections 207 are integrated into the box walls of the intercondylar box 206: a first projection 207a is integrated into the medial box wall 206a in the distal medial condyle portion 209a and a second projection 207b is integrated into the lateral box wall 206b in the distal lateral condyle portion 209b of the femoral component 200.

The projections 207a, 207b are configured to engage into corresponding sockets formed in a patient's femur for guiding the ceramic femoral component on the patient's femur in anterior-posterior direction and positioning the femoral component on the patient's femur (not shown). Said projections may also be described as positioning elements or anchoring elements.

As illustrated in Fig.2A, B each of the projection 207a, 207b extends inwardly from the distal portion 203 of the inner surface of the femoral component along and integrated into the respective medial box wall 206a and the lateral box wall 206b towards their upper edge.

The box wall projections 207a, 207b are of a semi-cylindrical shape, with a tapered, semi-conical (or round off) tip towards the upper end of the respective box wall 206a, 206b. The diameter of the box wall projections 207a, 207b is about 6 mm. The length of the box wall projections 207a, 207b depends on the length of the box walls 206a, 206b which is depending on the patient, but typically between 7 and 11 mm.

The box wall projections 207a, 207b are centered over the contact point of the femoral component with the tibial component when the contact point is 0° flexion (full extension) in vivo as defined in DIN EN ISO 21536..

Posterior cam 217 is disposed between the distal medial condyle portion 209a and distal lateral condyle portion 209b. Posterior cam 217 has a cylindric shape with notches for engaging with bars.

Three separate cement pockets 211, 212a, 212b are provided in the anterior portion 210 of the femoral component 200: one larger cement pocket 211 and two smaller cement pockets 212a, 212b. The two smaller cement pockets 212a, 212b are provided in the (lower) section 214a of the anterior portion 201 that is adjacent to the chamfer anterior portion 202 and the larger cement pocket 211 is provided in the upper section 214b of the anterior portion 201.

Two further cement pockets 213a, 213b are provided on the lateral and medial condyle spanning over anterior chamfer portion 202, distal portion 203, posterior chamfer portion 204 and posterior portion 205. Separate cement pockets (not shown) may also be provided in the posterior portion 205 which are important that the cement is not pushed out.

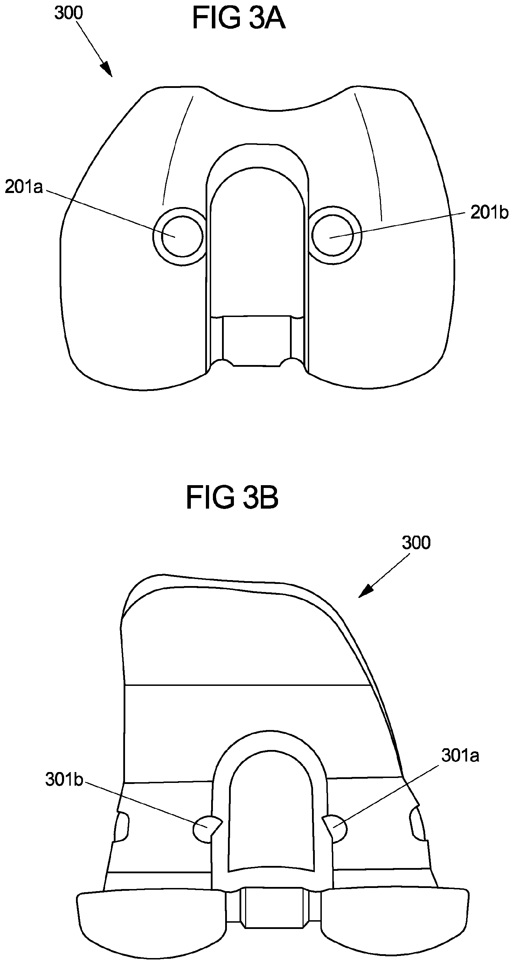

As described above in context with Fig. 1A-C the femur of the patient has to be prepared for receiving the ceramic femoral component. For this purpose, a femoral trial 300 as illustrated in Fig. 3A-B serves as a trial for testing the prepared knee just before implanting the final prosthetic femoral component 200.

The geometry of femoral trial 300 is similar, but not identical, in size and shape to prosthetic femoral component 200. The internal geometry of femoral trial 300 is smaller than of femoral component 200.

In addition, the femoral trial 300 comprises two through holes 301a, 301b for providing holes in the distal femur of the patient for guiding and receiving the box wall projections 207a, 207b of the femoral component 200. The position of the through hole 301a in the trial 300 corresponds to the position of the box wall projection 207a in the ceramic femoral component and the position of the through hole 301b corresponds to the position of the box wall projection 207b in the ceramic femoral component 200. The diameter of through holes is larger than the diameter of the semicircular box wall projections by approximately 0.4 mm.

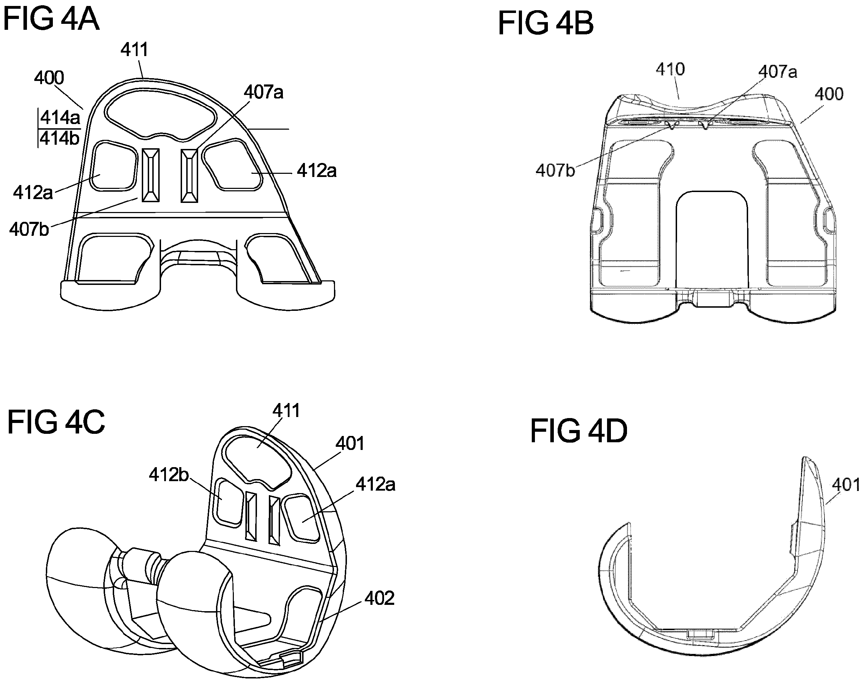

Figs. 4A-D show another embodiment of the femoral component 400 according to a second aspect.

As illustrated, the ceramic femoral component 400 comprises also a medial condyle section with a distal medial condyle portion, a lateral condyle section with a distal lateral condyle portion and an intercondylar space between the distal medial condyle portion and the distal lateral condyle portion Further details of the femoral component are described previously and are fully applicable.

The femoral component 400 has two projections or fins 407a, 407b that extend inwardly from the anterior portion 411 of the inner surface of the femoral component 400 for engaging into the patient's femur for positioning the femoral component on the patient's femur. Projections or fins 407a, 407b bridge or span over the previously described gap 102 between the femur bone and the femoral component (see Fig. 1A) and press into the bone of the patient's femur for positioning and anchoring the ceramic femoral component on the patient's femur.

Projections 407a, 407b are arranged in the lower section 414a of the anterior portion 401 in close proximity and perpendicular to the contact line (or bending line) between the anterior portion and the chamfer anterior portion 402.

Projections 407a, 407b are provided in the lower section 414a of the anterior portion between two cement pockets 412a, 412b. Specifically projections 407a, 407b are centered between the two smaller cement pockets 412a, 412b provided in the lower section of the anterior portion of the ceramic femoral component. Larger cement pocket 411 is arranged in the upper section 414b of the anterior portion 401 above projections 407a, 407b.

Projections 407a, 407b in the anterior portion 401 have a height between 1.1 mm and 1.3 mm and a length between 20 and 30 mm, and a width between 1.0 and 2.5 mm.

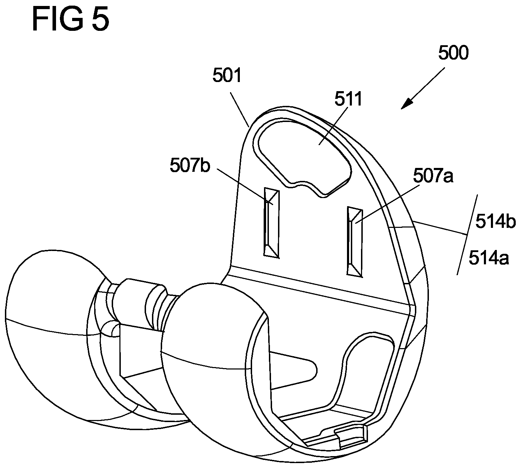

Fig. 5 shows another embodiment of the femoral component 500 according to a second aspect.

As illustrated in Fig. 5 two smaller cement pockets are replaced by projections 507a, 507b that extend inwardly from the anterior portion 501. Here, projections 507a, 507b are integrated in the anterior portion in an outer region of in the lower section 514a of the anterior portion 501. Larger cement pocket 511 is arranged in the upper section 514b of the anterior portion 501 above projections 507a, 507b.

The embodiments disclosed herein are for the purpose of providing an exemplary description of the present subject matter. They are, however, only exemplary and not exclusive, and the present subject matter may be embodied in various forms. Therefore, specific details disclosed herein are not to be interpreted as limiting the subject matter as defined in the accompanying claims.

### List of reference signs

- 100: femoral component 100,
- 101: patient's femur 101
- 102a,b: gaps between femoral component 100 fit onto patient's femur 101

- 200: femoral component
- 201: anterior portion
- 202: anterior chamfer portion
- 203: distal portion
- 204: posterior chamfer portion
- 205: posterior portion
- 206: intercondylar box
- 206a: medial box wall
- 206b: lateral box wall
- 207: projection
- 207a: first projection is integrated into the medial box wall 206a
- 207b: second projection is integrated into the lateral box wall 206b
- 208a: medial condyle section
- 208b: lateral condyle section
- 209a: distal medial condyle portion
- 209b: distal lateral condyle portion
- 210: trochlear or patella groove
- 211: larger cement pocket
- 212a: smaller cement pocket
- 212b: smaller cement pocket
- 213a: further cement pocket
- 213b: further cement pocket
- 214a: lower section of anterior portion 201
- 214b: upper section of anterior portion 201
- 215a: medial recess
- 215b: lateral recess
- 216: intracondylar space
- 217: posterior cam

- 300: trial for femoral component 200
- 301a: medial bore hole
- 301b: lateral bore hole

- 400: femoral component
- 401: anterior portion
- 406: intercondylar box
- 407a: first projection or fin integrated into the anterior portion 401
- 407b: second projection or fin integrated into the anterior portion 401
- 410: trochlear or patella groove
- 411: larger cement pocket
- 412a: smaller medial cement pocket
- 412b: smaller lateral cement pocket
- 414a: upper section of anterior portion 401
- 414b: lower section of anterior portion 401

- 500: femoral component
- 501: anterior portion
- 507a: first projection or fin integrated into the anterior portion 501
- 507b: second projection or fin integrated into the anterior portion 501
- 511: larger cement pocket
- 514a: lower section of anterior portion 501
- 514b: upper section of anterior portion 501

## Claims

1. A ceramic femoral component of a knee prothesis with an anterior end and a posterior end comprising
- an inner surface of the femoral component, and
- at least one, preferably at least two projections extending inwardly away from the inner surface of the femoral component for guiding the ceramic femoral component on the patient's femur in anterior-posterior direction and preventing tilting of the femoral component and positioning the ceramic femoral component on the patient's femur by formfit or pressfit.

2. Ceramic femoral component according to claim 1 **characterized in that** at least one, preferably at least two pressfitting projections are provided in the anterior portion or at least one, preferably at least two formfitting projections are provided in combination with the box the distal portion of the inner surface of the femoral component, in particular in the distal medial condyle portion and/or the distal lateral condyle portion of the femoral component.

3. Ceramic femoral component according to one of the preceding claims **characterized by**
- an inner surface of the femoral component including an anterior portion, an anterior chamfer portion, a distal portion, a posterior chamfer portion and a posterior portion,
- a medial condyle section with a distal medial condyle portion,
- a lateral condyle section with a distal lateral condyle portion,
- an intercondylar space between the distal medial condyle portion and the distal lateral condyle portion,
- an intercondylar box substantially disposed between the distal medial condyle portion and distal lateral condyle portion for filling the intercondylar space, the box including a medial box wall and a lateral box wall, the walls extending from the inner surface of the femoral component towards an upper end / upper edge of said wall,
- wherein each of the medial box wall and the lateral box wall comprise at least one projection for engaging into corresponding sockets formed in a patient's femur for positioning the femoral component on the patient's femur by medial and lateral formfit,
- wherein each of the at least one projection extends from the distal portion of the inner surface of the femoral component along the respective medial box wall and the lateral box wall towards the upper end (or edge) of the medial box wall and the lateral box wall.

4. Ceramic femoral component according to claim 3, **characterized in that** said projection is formed as one-piece with the respective medial box wall and lateral box wall.

5. Ceramic femoral component according to one of the preceding claims, **characterized in that** said projection has a angular or a cylindrical shape, in particular a semi-cylindrical shape, with a tapered, conical (or round off) tip, in particular semi-conical tip, towards the upper end of the respective box wall.

6. Ceramic femoral component according to one of the preceding claims, **characterized in that** said projection (or positioning element) has a dimension between 2 and 10 mm, preferably between 4 and 8 mm, more preferably between 5 and 6 mm.

7. Ceramic femoral component according to one of the preceding claims, **characterized in that** said projections extend in a perpendicular line from the distal portion of the inner surface of the femoral component towards the upper end (or edge) of the medial box wall and the lateral box wall.

8. Ceramic femoral component according to one of the preceding claims, **characterized by** a cam portion substantially disposed between the distal medial condyle portion and distal lateral condyle portion.

9. Ceramic femoral component according to one of the preceding claims, **characterized in that** the medial box wall and a lateral box wall of the intercondylar box are curved, oblique, straight or a combination thereof, preferably curved.

10. Ceramic femoral component according to one of the preceding claims **characterized by** at least one, preferably at least two, more preferably at least three cement pockets arranged in the anterior portion of the inner surface of the femoral component.

11. Ceramic femoral according to one of claims 1 or 2 comprising
- an innersurface of the femoral component including an anterior portion, an anterior chamfer portion, a distal portion, a posterior chamfer portion and a posterior portion, and
- at least one, preferably at least two projections extending from the anterior portion of the inner surface of the femoral component for engaging into the patient's femur by pressfit for positioning the femoral component on the patient's femur.

12. Ceramic femoral component according to claim 11, **characterized in that** said projections are provided on the anterior portion of the inner surface of the femoral component along a line that extends perpendicular (or vertically) from the contact line of the anterior portion and the anterior chamfer portion.

13. Ceramic femoral component according to one of the claims 11 to 12, **characterized in that** said projections are provided in a lower section of the anterior portion adjacent to the chamfer anterior portion of the inner surface of the femoral component between cement pockets, in particular between two cement pockets.

14. Ceramic femoral component according to one of the claims 11 to 13, **characterized in that** the projections in the anterior portion have a height between 0.5 mm and 10 mm, preferably between 1.00 mm and 5.0 mm, more preferably between 1.5 mm and 2.5 mm, a length between 5 and 50 mm, preferably between 10 and 40 mm, more preferably between 20 and 30 mm, and a width between 0.5 and 5.0 mm, preferably between 0.7 and 4.0 mm, more preferably between 1.0 mm and 2.5 mm.

15. Ceramic femoral component according to one of the claims 11 to 14, **characterized in that** no intercondylar box substantially is disposed between the distal medial condyle portion and distal lateral condyle portion for filling the intercondylar space.
